# EUROPEAN PATENT SPECIFICATION

(11) **EP 2 121 052 B1**
(45) Date of publication and mention of the grant of the patent: **20.04.2016**
(21) Application number: 06817786.4
(22) Date of filing: 22.12.2006
(51) Int. Cl.: A61L 27/46, A61L 27/52, A61L 27/56

(54) **PRECURSOR FOR THE PREPARATION OF A PASTY BONE REPLACEMENT MATERIAL BY ADMIXTURE OF A LIQUID**
VORLÄUFER ZUR HERSTELLUNG EINES PASTENFÖRMIGEN KNOCHENERSATZMATERIALS DURCH MISCHUNG MIT EINER FLÜSSIGKEIT
PRÉCURSEUR POUR LA PRÉPARATION D'UN MATÉRIAU PÂTEUX DE REMPLACEMENT D'OS PAR ADJONCTION D'UN LIQUIDE

(43) Date of publication of application: 25.11.2009
(73) Proprietor: Mathys AG Bettlach, 2544 Bettlach (CH)
(72) Inventor: BOHNER, Marc, CH-2540 Grenchen (CH); RIZZOLI, Giancarlo, CH-4500 Solothurn (CH)
(74) Representative: Lusuardi, Werther
(86) International application number: PCT/CH2006/000736
(87) International publication number: WO 2008/077257

(56) References cited:
- EP-A- 0 416 398
- WO-A-93/20858
- US-A1- 2003 199 615
- US-A1- 2004 062 816
- US-A1- 2005 136 122

## Description

The invention relates to a precursor for the preparation of a pasty bone replacement material by admixture of a liquid according to the preamble of claim 1.

A number of bone replacement material prepared from solid, dry precursors by admixing of a liquid are known. However, all of the known precursor materials are either non-sterile or are degraded in their molecular structure by the sterilization process. In particular the usual dry autoclaving (e.g. for 120 minutes at 170°C-WHO 1986) leads to the destruction of most hydrogels used in such bone replacement materials.

Materials which can be injected are also known. For example, hydraulic calcium phosphate cements consist of one or several calcium phosphate powders and an aqueous solution. Upon mixing, a paste is formed. This paste can be injected and hardens within (typically) 5 - 20 minutes. Unfortunately, the resulting hardened paste is still brittle and can only be resorbed layer-by-layer, i.e. much slower than the pastes described in the present invention. Other injectable pastes consist of non cementitious mixtures of microsized calcium phosphate particles and an aqueous solution. Again, resorption only occurs layer-by-layer. A third alternative is to combine spherical particles (larger than about 0.1 mm) and a low-viscosity hydrogel. These mixtures are injectable, and have a well distributed resorption due to the presence of gaps between the spherical particles, but these mixtures are not kneadable and present a low cohesion.

From EP-A 0 416 398 a paste for bonding a granular prosthesis is known which comprises an aqueous solution of pullulan, glycol chitin , carboxmethyl chitin or pectin. From US 2004/0062816 a bone repair material is known comprising a porous, resorbable particulate and a resorbable carrier gel for suspending the particulate. Both of these prior art documents do not disclose a precursor for the preparation of a pasty bone replacement material by admixture of a liquid, and where the precursor is sterile for surgical use.

In the following text, the term "particle" includes any three-dimensional body, regardless of its dimensions, especially the small parts commonly known as "granules" or "grains". The sphericity S of the particles (or spherical relationship) is defined as the ratio of Dₘₐₓ/Dₘᵢₙ between the largest diameter Dₘₐₓ and the smallest diameter Dₘᵢₙ of the individual particles. Fully spherical particles therefore have a sphericity S = 1.00.

This discussion regarding current standards of technology is used only to explain the environment of the invention and does not mean that the standards of technology quoted here were actually published or publicly known at the time of this registration or its priority. From EP-A 0 416 398 a paste for bonding granular bone prosthesis is disclosed which comprises an aqueous solution containing pullulan, glycol chitin, carboxymethyl chitin and pectin.

This invention is meant to provide a remedy for this situation. The invention is based on the problem of creating a precursor which overcomes the disadvantages listed above. The invention solves this task with a precursor which has the characteristics of claim 1.

It is an object of the invention to provide a solid precursor for the preparation of a pasty bone replacement material by admixture of a liquid, whereby said precursor remains stable prior to use and in particular retains its molecular integrity to a high degree.

The advantages of the invention are the following:
- Large versatility since the dry precursor can be mixed with many different liquids such as blood, platelet-rich plasma, antibiotic solution or bone marrow;
- Very good handling (fast obtention of a pasty material);
- Optimum resorption of the kneadable bone replacement material obtained from the precursor;
- good optical appearance (no color heterogeneities due to the presence of solid particles having a inadequate size); and
- easy production (due to a sufficiently large size of the solid particles allowing automatic weighing.

In a special embodiment the precursor has been obtained by wet autoclaving and subsequent drying of at least said swellable substance.

A hydrogel is present when a solid substance is hydrated via a liquid phase, changing and increasing the viscosity of the liquid phase, i.e. jellying or coagulating the liquid phase. Some hydrogels are rather elastic others are rather plastic (e.g. sodium hyaluronate). An elastic hydrogel can be destroyed with shear forces, contrary to a plastic (deformable) hydrogel.

The solid particles are of ceramic or mineral nature and they may contain calcium.

The solid particles may alternatively be based on bioglass(es).

The hydrogel matrix can consist of oligomeric or polymeric parts or of a combination of the two.

The solid particles and said swellable substance are present as a mixture.

The discrete particles of said swellable substance have a mean diameter of at least 50 µm. The discrete particles of said swellable substance have a mean diameter of less than 1000 µm.

The swellable substance comprises:
a polyamino-acid or its derivatives; or
a polysaccharide and their derivatives; preferably glycosaminoglycane or alginate; or hyaluronic acid, sodium hyaluronate, chondroitinsulfate, dermatansulfate, heparansulfate, heparine or keratansulfate.

The swellable substance may consist of either a glycosaminoglycane or a proteoglycane or a mixture of those two substances. The glycosaminoglycane may be a hyaluronic acid, chondroitinsulfate, dermatansulfate, heparansulfate, heparine or keratansulfate.

In a further embodiment the hydrogel is hyaluronic acid. The hyaluronic acid consists of glucuronic acid and acetylglucosamine which create the disaccharide hyaluronic acid. The hyaluronic acid has a fibrous, non-branched molecular structure and therefore results in highly viscous liquid solutions. The hydrogel may also be in the form of sodium hyaluronate (NaHyA).

In a further embodiment the swellable substance is of fully synthetic origin. This eliminates the danger of transferring diseases due to the absence of possible pathogenic agents such as proteins, germs, viruses or bacteria as compared to precursors of natural origin.

Alternatively the swellable substance may consist of a biotechnologically generated substance (e.g. fermentation).

The MW of the swellable substance is - after sterilization - larger than 300'000 Dalton and preferably larger than 500'000 Dalton. In a further embodiment the MW of the swellable substance is larger than 1'000'000 Dalton and preferably larger than 1'500'000 Dalton. Concerning the MW, it is important to know that it is often calculated based on viscosity data, in particular the "intrinsic viscosity" of the polymer. It may be advantageous therefore to specify the intrinsic viscosity of the polymer rather than its MW. An initial intrinsic viscosity larger than 2.0 m³/kg, preferably larger than 2.5 m³/kg appears to be the minimum required to obtain a good product after sterilization. After sterilization, the intrinsic viscosity should preferably be superior to 1.3 m³/kg.

In a further embodiment said swellable substance has an intrinsic viscosity of at least 1.3 m³/kg, preferably at least 1.4 m³/kg after sterilization. Preferably at least 80 % of said intrinsic viscosity is reached within 5 minutes, preferably within 2 minutes after the start of mixing.

In a further embodiment said discrete particles of said swellable substance have a sphericity S smaller than 5, preferably smaller than 2.

In a further embodiment the precursor further comprises a drug having an active effect on bone metabolism, preferably osteoinductive substances, drugs against osteoporosis or antimicrobial drugs. Examples for osteoinductive substances are: morphogenetic proteins and growth factors; examples for drugs against osteoporosis are: biphosphonates and parathyroid hormone; an example for an antimicrobial drug is gentamycin sulfate.

In a further embodiment the solid particles have at least a partially porous structure. The pore size of the solid particles is preferably between 10 nanometers and 500 micrometers.

Preferably at least 50% of the solid particles have a pore size between 100 and 500 micrometers. This guarantees optimum pore size distribution and the growth of autogenous tissue into the pores.

In a further embodiment the porosity of the solid particles is between 60 and 90 percent, preferably between 68 and 84 percent. This ensures that autogenous tissue is able to grow into a large volume share of solid particles.

The average diameter of the solid particles is preferably between 100 and 500 micrometers. The advantage of this is the fact that the paste obtained by admixing the precursor with a liquid gets a smooth consistency. In addition, the risk of irritation within the tissue surrounding the particles is practically non-existent, if the diameter of the particles is not smaller than 100 micrometers.

It is also possible to mix the solid particles with two different size populations, e.g. particles with an average diameter between 125 and 250 micrometers and particles with an average diameter between 500 and 710 micrometers or an average diameter between 0.5 and 5.6 mm. This has the advantage that it guarantees the compactness of the bone replacement material. The interstitial pore volume (pore dead volume) which results from the use of large-grain material can thus be reduced to a minimum. It is also possible to affect the degradation period of the bone replacement material through the use of solid particles of various sizes. (smaller particles are resorbed faster than larger particles).

The specific gravity of said solid particles may be between 0.5 and 1.0 g/ccm. Alternatively to the spherical form the solid particles may have also a non-spherical shape. The solid particles may have a specific surface area (SSA) of larger than 0.01 m²/g, preferably larger than 0.1 m²/g. The solid particles may have also a specific surface area (SSA) of larger than 5 m²/g, preferably larger than 50 m²/g. A high SSA is advantageous for drug delivery purposes. Drugs trapped in the porous structure diffuse out at a very slow rate, typically over days or even weeks.

In a special embodiment the discrete particles of said swellable substance have a mean volume of at least 3 ·10⁻⁶ mm³, preferably of at least 65 ·10⁻⁶ mm³. In another embodiment the discrete particles of said swellable substance have a mean volume of maximum 4.2 mm³, preferably of maximum 0.5 mm³.

In a further embodiment the precursor contains less than 10 weight-percent, preferably less than 1 weight-percent of gelatin. Most preferably the precursor is free of gelatin.

In a further embodiment the precursor contains a radiopacifier. The use of a radiopacifier is beneficial for certain application, such as vertebroplasty or kyphoplasty. It allows a better visualization of the position of the bone substitute during and after insertion into the bone defect. The radiopacifier may be selected from the following group: tantalum powder, tungsten powder, titanium powder, zirconium oxide powder, bismuth oxide powder, iode-based liquid. A suitable iode-based liquid is iopamidol.

To improve the biological efficiency, the paste produced by mixing the various components of the precursor according to the invention should preferably present large domains between the solid particles to allow a rapid cell invasion through the hydrogel (present between the solid particles) and hence enable a fast ingrowth of bone within the bone substitute (because the hydrogel is resorbed or removed within a few days).

The term "Non-spherical" describes any particle shape which is significantly different from a spherical shape. One variant of the invention uses solid particles with an angular shape. "Angular" describes those particles which have individual edges, especially those which are visible with the naked eye, i.e. which are at least 0.1 mm in size. Compared to round particles, these results in an increase to the particle surface, while the average particle diameter remains the same. This causes the adhesive interaction between the solid particles and the hydrogel to be increased, guaranteeing the mouldability of the bone-replacement material without the need for increasing the quantity of hydrogel used or its concentration.

There is also a special embodiment where the solid particles have a spherical relationship S = Dmax/Dmin between the largest diameter Dmax and the smallest diameter Dmin of the individual particles, which is larger than 1.2 and preferably larger than 1.5. The value of S should be larger than 3 and preferably larger than 5. Preferably at least 60 % and typically at least 80 % of the solid particles should be of a non-spherical shape.

Alternatively, it might be of interest to provide an injectable paste. For that purpose, it is important to use round particles. Mixtures of various particle sizes can lead to a more compact paste that is also better injectable.

To provide a fast bone ingrowth and resorption of the solid particles, at least 60 % and typically at least 80 % of the solid particles should be of a non-spherical shape.

In a further embodiment the packed bulk density of the solid particles, e.g. in form of calcium containing, porous ceramic particles is preferably between 0.5 and 1.0 g/ccm.

Particles with a high specific surface area (SSA) are characterized by the presence of numerous nanosized pores or by a high surface corrugation. As a result, such particles are of great interest for drug delivery applications: drugs entrapped in the nanopores diffuse at a very low rate, hence providing an excellent drug delivery system. Particles obtained at high temperature (e.g. β-tricalcium phosphate, α-tricalcium phosphate, tetracalcium phosphate, calcined bone) have normally a very low SSA, typically in the range of 0.001 to 1 m²/g. Particles synthesized at or close to room temperature present generally much higher SSA. For example, particles obtained by purifying bone chips (extraction of organic matter) have a SSA typically in the range of 50-100 m²/g. Particles obtained by hydraulic reactions (e.g. calcium phosphate cement) have a SSA in the range of 10-200m²/g depending on the composition.

In a further embodiment the solid particles comprise a calcium phosphate which is characterized by a molar Ca/P relationship between 1.0 and 2.0. Preferably the ceramic particles comprise a calcium phosphate which is characterized by a molar Ca/P relationship between 1.45 and 1.52.

The calcium phosphate may be selected from the following group: Dicalcium phosphate dihydrate (CaHPO₄ x 2 H₂O), dicalcium phosphate (CaHPO₄), alpha-tricalcium phosphate (alpha-Ca₃(PO₄)₂), β-tricalcium phosphate (β-Ca₃(PO₄)₂), calcium-deficient hydroxyapatite (Ca₉(PO₄)₅(HPO₄)OH), hydroxyapatite (Ca₁₀(PO₄)₆OH)₂), carbonated apatite (Ca₁₀(PO₄)₃(CO₃)₃(OH)₂), fluoro apatite (Ca₁₀(PO₄)₆(F,OH)₂), chloro apatite (Ca₁₀(PO₄)₆(Cl,OH)₂), whitlockite ((Ca,Mg)₃(PO₄)₂), tetracalcium phosphate (Ca₄(PO₄)₂O), oxyapatite (Ca₁₀(PO₄)₆O), β-calcium pyrophosphate (β-Ca₂(P₂O₇), α-calcium pyrophosphate, gamma-calcium pyrophosphate, octocalcium phosphate (Ca₈H₂(PO₄)₆ x 5 H₂O). The various calcium phosphate materials may also be doped with elements such as Na, Cl, F, S, C, Sr, Mg, Zn, Si, Fe, Li, K or Ag.

In a further embodiment the solid particles comprise a mixture of different calcium phosphates. The advantage of such a mixture lies in the control of the resorption period. Due to the differing resorption behaviors of the mixture components, faster bone growth into the cavities of components with faster resorption times can be facilitated.

Alternatively the solid particles may comprise a calcium sulphate (anhydrous, hemihydrate, dehydrate, and their polymorphs), a calcium carbonate (calcite, aragonite or vaterite), bioglass or a mixture of different calcium phosphates, calcium sulfates, calcium carbonates and/or calcium-containing bioglass. The advantage of such a mixture lies in the control of the resorption period. Due to the differing resorption behaviors of the mixture components, faster bone growth into the cavities of components with faster resorption times can be facilitated.

Preferably, the maximum amount of residual water present in the solid precursor (expressed by the loss on drying at 105°C) is smaller than 5%, preferably smaller than 2%. The presence of absorbed water triggers the decomposition of the hydrogel and hence the residual humidity in the dry product should be kept as low as possible.

The preparation of a bone replacement material is obtained by admixing a liquid to the precursor. The following liquids are suitable for that purpose: pure water, sterile demineralized water, an aqueous solution, a sterile saline solution, sterile Ringer solution, an antimicrobial drug solution - preferably an antibiotic solution - or a solution containing osteoinductive substances - preferably bone morphogenetic proteins such as BMP2 and BMP7 or growth factors - and/or drugs against osteoporosis - preferably bisphosphonates and parathyroid hormone. The surgeon has the possibility to replace the sterile solution with blood, blood extract (e.g. serum, platelet rich plasma), bone marrow, bone marrow extract, or any human extract having a beneficial effect on bone formation.

The solid precursor has to be sterile for surgical use. There are two sterilization approaches that can be used: (i) combine two sterile products and package them in an aseptic environment, or (ii) prepare the solid precursor and then sterilize it. The first approach is at first sight the easiest, but for production cost reasons, the second approach is nowadays the best approach. Therefore, the bone replacement material and the swellable substance must be simultaneously sterilized. Among the various sterilization techniques that can be used for solids (gamma irradiation, plasma, ethylene oxide, autoclaving, hot air), autoclaving is the best possible technique due to (i) the good homogeneity of the sterilization method, (ii) an absence of toxicity, and (iii) the ability to retain the molecular integrity of the powder substance.

Preferably, autoclaving (= steam sterilization) is done in such a way that it does not lead to a molecular weight (MW) loss of the hydrogel greater than 70%. Autoclaving may be performed at various temperatures for various durations. In fact, higher temperatures require shorter sterilization times (see Chapter 4 "Verfahren zur Verminderung der Keimzahl" of "Sterilisation, Desinfektion, Konservierung, Keimidentifizierung, Betriebshygiene (edition 1988).

Keeping a given autoclaving duration (e.g. 18 min), a too low temperature is not able to sterilize the sample, whereas a too large temperature drastically destroys the polymer. As a result, an intermediate temperature is ideal. For example, it could be observed with sodium hyaluronate and β-TCP granule mixtures that a temperature of 121°C (18 min autoclaving) was too low (only partly sterile) whereas a temperature of 128°C (18min autoclaving) was too high. A temperature of 125°C for a duration of 18 min appeared therefore to be optimal.

Preferably the autoclaving does lead to a decrease of the MW of said swellable substance of minimum 30 % and of maximum 70 %. The autoclaving may be performed at a temperature in the range of 110° to 140°C, preferably of 121° - 128°C. The drying of the precursor may be obtained by the action of dry air, vacuum, freeze-drying and/or a desiccating agent. Preferably the loss on drying at 105 °C of said precursor is smaller than 5%, preferably smaller than 2%.

In a preferred embodiment the ratio between the dry weight of the swellable substance and the liquid is in the range of 0.001 and 0.500. Higher concentrations lead to higher costs and lower concentrations do not lead to the desired plastic and firm type material. Preferably the ratio between the dry weight of the swellable substance and the liquid is in the range of 0.03 and 0.09.

In a further embodiment the weight relationship between the hydrated hydrogel and the solid particles is larger than 0.2, preferably larger than 0.6. In another embodiment the weight relationship between the hydrated hydrogel and the solid particles is smaller than 4, preferably smaller than 2.

The precursor can be made available in form of a kit comprising the precursor together with a liquid suitable for admixing to said precursor in order to convert the resulting mixture into a kneadable mass for bone replacement. Preferably the bone replacement material product is presented to the surgeon as a kit consisting of a sterile powder [e.g. β-TCP granules + NaHyA powder] and a sterile liquid, e.g. deionized water or saline solution.

Experimentally, so-called cohesion tests have been performed with pastes produced with various NaHyA particle sizes. The "cohesion" of a paste is defined as the ability of a paste to stay in one piece when placed into contact with an aqueous solution. This property can be measured by dipping a paste in an aqueous solution (e.g. 4 minutes after the start of the preparation) and measuring the weight loss of the paste over time (Bohner et al, Eur Cells Mater, 2006). Two approaches can be used to quantify the results: measure how long it takes until a known amount of weight has been lost (e.g. -0.3g) or how much material has been lost within a given time period (e.g. between the time points 10 and 30 minutes of the measurement)

The precursor according to the invention has to be mixed with a liquid to obtain a pasty material. It has been found that the viscosity of the resulting paste is not only a function of the concentration and molecular weight of the swellable substance, but also of the kinetics of the dissolution of the swellable substance.

Since the dissolution of the swellable substance is a function of the interface area between the swellable substance and liquid, small particles dissolve much faster than large particles. As a result, relatively small particles are preferred to large particles.

Small particles do not flow very well. Therefore, big particles should be preferred to small particles for production purposes because it is easier to automatically weigh them. Similarly, round particles flow much better than fibers, i.e. round and large particles (>20 - 50 microns in diameter) are the most adapted to production purposes.

Furthermore, the particles of the swellable substance tend to shrink during autoclaving and subsequent drying. The resulting change of particle density may provoke a change of the optical appearance of the hydrogel. For example, the color of NaHyA particles changes from white/translucent to yellow. As calcium phosphate particles are generally white, the presence of yellow particles in the product has a negative effect on its aesthetic properties. This effect is a function of the hydrogel particle size: particles smaller than about 0.5mm are too small to be detected by eye sight, and hence the product appearance is maintained (no apparent heterogeneities in the product), i.e. the hydrogel particles have a diameter smaller than 1 mm, preferably smaller than 0.5 mm.

### A BRIEF DESCRIPTION OF THE DRAWINGS

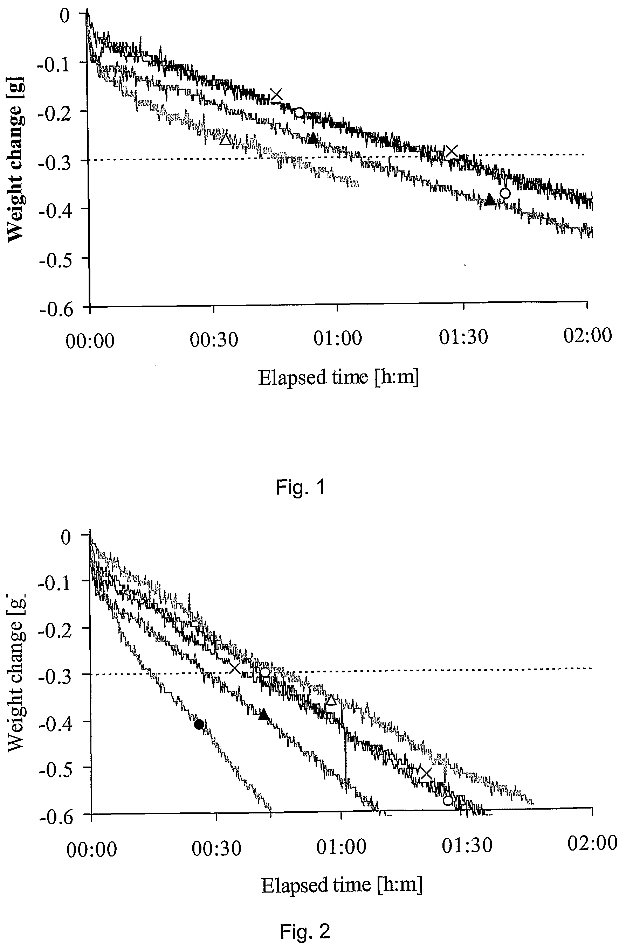

Figure 1: representative curves obtained in the cohesion test with a 1.76MDa NaHyA powder (intrinsic viscosity: 2.68m³/kg). The particle diameter was: (▲) d < 0.125mm; (o) 0.125 < d < 0.250mm; (x) 0.250 < d < 0.500mm; (▲) 0.500 < d < 1.000mm. Putty composition: 0.94g 0.125-0.250mm β-TCP granules, 0.94g 0.50-0.71 mm β-TCP granules, 102mg NaHyA, 1.6mL deionized water.
Figure 2: representative curves obtained in the cohesion test with a 1.1 MDa NaHyA powder (intrinsic viscosity: 1.64m³/kg). The particle diameter was: (▲) d < 0.125mm; (o) 0.125 < d < 0.250mm; (x) 0.250 < d < 0.500mm; (▲) 0.500 < d < 1.000mm; and (●) 1.0 < d < 2.0mm. Putty composition: 0.94g 0.125-0.250mm β-TCP granules, 0.94g 0.50-0.71 mm β-TCP granules, 102mg NaHyA, 1.6mL deionized water.

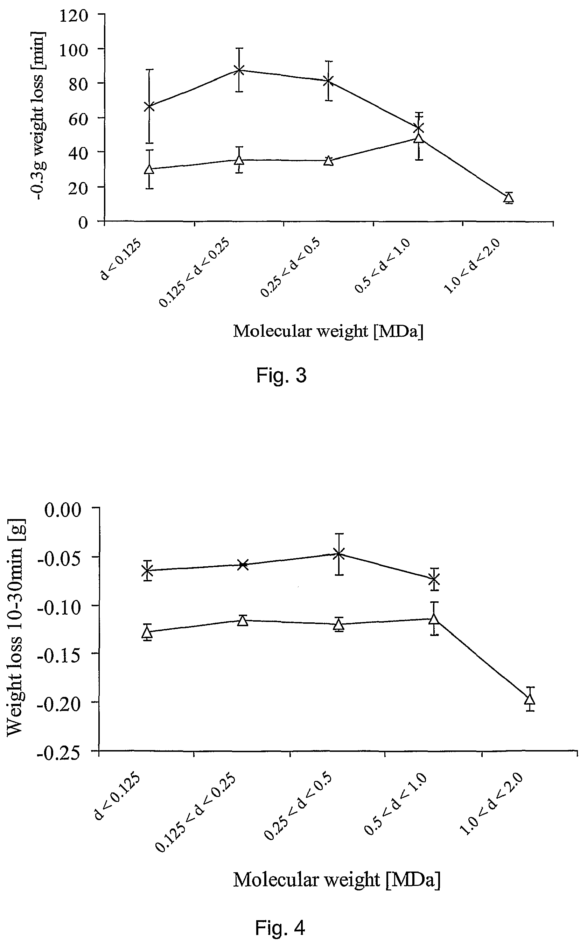

Figure 3: Effect of particle size on the average duration until the weight loss amounted -0.3g; (x) 1.76MDa powder (intrinsic viscosity: 2.68m³/kg); (▲) 1.1 MDa powder (Intrinsic viscosity: 1.64m³/kg).
Figure 4: Effect of particle size on the weight loss between 10 and 30min; (x) 1.76MDa powder (intrinsic viscosity: 2.68m³/kg); (▲) 1.1 MDa powder (Intrinsic viscosity: 1.64m³/kg).

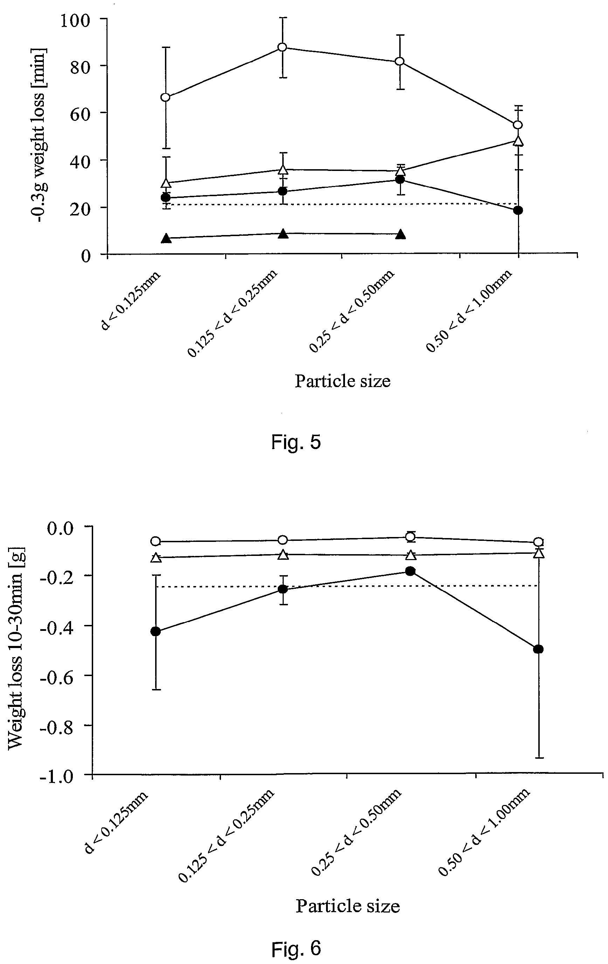

Figure 5: Effect of NaHyA molecular weight, particle size and sterilization on the duration until a 0.3g weight loss was measured during cohesion testing. (o) 1.76MDa NaHyA, before autoclaving; (●) 1.76MDa NaHyA, after autoclaving; (▲) 1.1 MDa NaHyA, before autoclaving; (▲) 1.1 MDa NaHyA, after autoclaving (only one measurement). The red dotted line represents the value obtained with a 0.67MDa NaHyA powder (intrinsic viscosity: 1.3m³/kg).
Figure 6: Effect of NaHyA particle size and sterilization on the cohesion weight loss measured between 10 and 30min of testing. (o) 1.76MDa NaHyA, before autoclaving; (●) 1.76MDa NaHyA, after autoclaving; (▲) 1.1 MDa NaHyA, before autoclaving. The red dotted line represents the value obtained with a 0.67MDa NaHyA powder (intrinsic viscosity: 1.3m³/kg). No values were reported for 1.1 MDa NaHyA powder after autoclaving due to the fact that the paste fell off within the first 10 minutes.

The results obtained with 4 to 5 size fractions of 2 different NaHyA powders are shown in Figures 1 - 4. The results suggest that a particle size larger than 1 mm is not adequate. After autoclaving the samples at 125°C for 18 min, and drying them at 60°C for 4 hours, the sample cohesion is decreased due to a decrease of the average MW of NaHyA (Fig 5 - 6). Interestingly, the results suggest that the size fraction 0.5 - 1.0 mm of the 1.76MDa NaHyA leads to worse results than the other 3 tested fractions. To summarize, these results show that the hydrogel particle diameter should be lower than 1 mm, possibly lower than 0.5 mm.

The invention and further developments of the invention are explained in more detail in the following examples:

### Example 1

### A) Obtaining a sterilized sodium hyaluronate

An aqueous solution of NaHyA having a MW of 1428 kDa and a particle diameter of 0.125 to 0.500 mm was autoclaved for 18 minutes at 125°C. By the autoclaving the MW of the NaHyA was reduced from originally 1400 kDa down to 800 to 1000 kDa (as measure by viscosimetry). The reduction of the MW had no negative effect on the qualities. Drying after wet autoclaving was done in dry air in the presence of P₂O₅ powder under sterile conditions. The sterility was provided by two steam-permeable membranes used to package the material before autoclaving.

### B) Obtaining a bone-replacement material of putty consistency

0.12 g of the obtained dried NaHyA (according to step A), 1.1 g of β-tricalcium phosphate powder (with a diameter in the range of 0.125 - 0.500 mm and a specific surface area of 0.01 - 0.30 m²/g) and 1.1 g of β-tricalcium phosphate powder (with a diameter in the range of 0.500 - 0.700 mm and a specific surface area of 0.01 - 0.30 m²/g) were mixed with 2 ml of sterile water with a spatula for 60 second. The β-tricalcium phosphate powders had a porosity of 60 %.

Two minutes after the start of mixing, a slightly elastic and kneadable mass was obtained. This paste was then kneaded to form a long and thin "worm" and inserted into a cancellous bone void resulting from a tibial plateau fracture. The void entry was then closed with the periosteum. Two and a half months after surgery, x-ray pictures demonstrated the presence of new bone in the defect and the start of the resorption process of the β-TCP granules. Full weight bearing could again be applied on the tibia.

### Example 2

A mixture of 24g of porous and angular granules of dicalcium phosphate DCP (with an approximate size of 500 micrometers, a sphericity degree of S = 3.1 and a specific surface area close to 7 m²/g) and 1.4 g chondroitin sulfate with a MW of 535 kDa was sterilized by autoclaving for 18 minutes at 125°C. Drying after wet autoclaving was done by freeze-drying under sterile conditions.

The sterile dry mixture was mixed with 25 mL of bone marrow aspirated from the pelvic bone of a 10-year old boy. The resulting mixture was kneaded in a sterilized bowl with a sterilized spatula for 1.5 minutes. Two minutes after the start of mixing, a slightly elastic and kneadable mass was obtained. This paste was then inserted into a cyst present in the humerus of the boy. The void entry was then closed with the periosteum. Six weeks after surgery, x-ray pictures demonstrated the presence of new bone in the defect and the start of the resorption process of the DCP granules. No empty void could be detected which could suggest the formation of a new cyst.

### Example 3

A mixture of 0.3g of 0.2 - 0.3 mm porous and spherical granules of calcium deficient hydroxyapatite having a specific surface area of 30 m²/g) and 0.3 g of 0.5 - 0.7 mm porous and spherical granules of calcium deficient hydroxyapatite having a specific surface area of 30 m²/g) was mixed with 50 mg of biotechnologically generated hydroxypropylmethyl cellulose with a MW of 900 kDa.

This mixture was sterilized by autoclaving for 18 minutes at 125°C. Drying after wet autoclaving was done by the action of dry air under sterile conditions.

Then, 0.1 mL of 5 weight percent gentamicin sulfate solution were added to the dried mixture and thoroughly mixed for 2 minutes. The resulting kneadable material was highly suitable as a plastic bone-replacement material and as a gentamicin delivery system.

### Example 4

0.2 g of sodium alginate (MW = 50 - 500 kDa / particle diameter < 0.71 mm) and 2.5 g of spherical granules of carbonated apatite (with a grain size of 200 - 300 microns and a specific surface area of 80 m²/g) were mixed and sterilized by autoclaving for 18 minutes at 125°C. Drying after wet autoclaving was done by the action of vacuum under sterile conditions.

Then 2.0 g of sterile Ringer solution were stirred into this dried mixture. This resulted in a kneadable material which was able to be used as a plastic bone-replacement material.

### Example 5

0.18 g of NaHyA (MW = 1.1 - 1.3 million Dalton), 2.5 g of spherical granules of carbonated apatite (with a grain size of 200 - 300 microns and a specific surface area of 80 m²/g), 1.0 g of tantalum powder (0.5 mm in diameter) and 1.5 g of porous and angular granules of β-tricalcium phosphate (ß-TCP) having a grain size of 125 to 500 micrometers, a sphericity of S = 2.5 and a specific surface area in the range of 0.01 - 0.30 m²/g) were mixed thoroughly and sterilized by autoclaving for 18 minutes at 125°C. After drying of the sterile mixture 0.5 ml of platelet-rich plasma under sterile conditions an amount of 1.5 ml of sterile deionized water were then stirred into this mixture. After thorough mixing, this resulted in an excellent plastic kneadable material which was able to be used as a plastic bone-replacement material.

### Example 6

0.18 g of NaHyA (MG = 1.1 - 1.3 million Dalton), 1.0 of porous and angular granulates of β-tricalcium phosphate (with a grain size of 500 to 700 micrometers, a sphericity degree of S = 2.9 and a specific surface area of 0.01 - 0.30 m²/g) and 1.5 g of porous and angular granulates of β-tricalcium phosphate (with a grain size of 125 to 500 micrometers, a sphericity of S = 2.5 and a specific surface area of 0.01-0.30 m²/g) were mixed thoroughly and sterilized by autoclaving for 18 minutes at 125°C. After drying of the sterile mixture (under sterile conditions) 2 ml of fresh blood were then stirred into this mixture. After thorough mixing, this resulted in an excellent plastic kneadable material which was able to be used as a plastic bone-replacement material.

### Example 7

### A) Manufacture of powder

A mixture of 6.6g β-TCP spherical granules with a size of 0.125 - 0.500 mm and a specific surface area of 0.01 - 0.30 m²/g)) and 0.27g NaHyA (MW = 1100 kDa) was autoclaved for 18 minutes at 125°C.

To make sure that autoclaving is effective and that the mixture stays sterile after autoclaving, the mixture was packaged twice in a blister package closed with a paper cover. The latter cover is permeable for steam, but not for germs.

After drying, the double blister package was packaged in an aluminum peel pouch to prevent humidity to decompose NaHyA during shelf life.

### B) Manufacture of liquid

6 mL of sterile Ringer solution were filled under aseptic conditions into two blister packages closed with an aluminum-coated membrane. The solution was then gamma irradiated with 25 - 42kGray to sterilize it.

### C) Use of the kit

The product kit consisted of a peel pouch containing the dry component (NaHyA and β-TCP granule) and the wet component. The kit was opened by a nurse in the surgical room. The peel pouch containing the dry component was opened above the sterile surgical table to drop the double-blister package onto the latter table. Afterwards, the surgeon opened both blister packages of the dry component, and placed the second (inner) blister package containing the powder/granule mixture on the sterile surgical table. The nurse opened the double blister containing the solution above the sterile surgical table and dropped the inner blister onto the table. The surgeon opened the latter blister, poured the liquid into the blister containing the powder/granules, and using a sterile metallic spatula, mixed the two components for one minute. Afterwards, the surgeon took the resulting paste in the fingers and kneaded it.

### Example 8

### A) Manufacture of powder

6.6 g of spherical β-TCP particles [with a diameter of 300 +/- 50 microns, an apparent density larger than 80% of the theoretical density (3.1 g/cc) and a specific surface area of 0.01 - 0.30 m²/g] and 0.36g NaHyA (MW = 1429 kDa / particle diameter . 0.125 to 0.500 mm) were packaged twice in a humidity-permeable blister and autoclaved for 18 minutes at 125°C. The sample was then freeze-dried until constant weight was reached. The external package was then removed and the inside part (humidity permeable blister) was dropped in a laminar flow bench and packaged in a sterile humidity-impermeable blister.

### B) Manufacture of liquid

6 mL of sterile distilled water were filled under aseptic conditions into the blister package obtained in step A, and the latter package was closed with an aluminum-coated membrane. The solution was then gamma irradiated with 25 - 42kGray to sterilize it.

### C) Use of the kit

According to example 7.

### Example 9

2 g of freezed-dried demineralised cortical allograft bone of particle size ranging from 250 - 420 microns was added to 93.6mg of sodium hyaluronate powder (0.1 - 0.5mm in diameter; 1.8MDa molecular weight, 2.7 m³/kg intrinsic viscosity), packaged twice in a steam permeable packaging material, and autoclaved for 18 minutes at 125°C. The sample was then dried for 4hours at 60°C, and packaged in a steam-proof package. This solid precursor was then mixed with 4.7mL whole blood from the patient during 2 minutes using a spatula to obtain a malleable putty with excellent formability properties.

## Claims

1. Precursor for the preparation of a pasty bone replacement material by admixture of a liquid, said precursor comprising
a) a biocompatible substance swellable by the action of water or of an aqueous solution thereby forming a hydrogel; and
b) solid particles made of a substance which is suitable as a bone replacement material,
**characterized in that**
c) the precursor is sterile for surgical use;
d) said swellable substance is in the form of discrete particles having a mean diameter in the range of 50 µm to 1000 µm;
e) said solid particles and said swellable substance are present as a mixture;
f) said solid particles are of ceramic or mineral nature;
g) the average diameter of said solid particles is between 100 and 500 micrometers;
h) the MW of said swellable substance is - after sterilization - larger than 300'000; Dalton; and
i) said swellable substance comprises:
a polyamino-acid or its derivatives; or
a polysaccharide and their derivatives; preferably glycosaminoglycane or alginate; or
hyaluronic acid, sodium hyaluronate, chondroitinsulfate, dermatansulfate, heparansulfate, heparine or keratansulfate.

2. Precursor according to claim 1, wherein said precursor has been obtained by wet autoclaving and subsequent drying of at least said swellable substance.

3. Precursor according to claim 1 or 2, wherein said solid particles contain calcium.

4. Precursor according to one of the claims 1 to 3, wherein said swellable substance has an intrinsic viscosity of at least 1.3 m³/kg.

5. Precursor according to one of the claims 1 to 4, wherein said solid particles are based on bioglass(es).

6. Precursor according to one of the claims 1 to 5, wherein said discrete particles of said swellable substance have a sphericity S smaller than 5.

7. Precursor according to claim 6, wherein said discrete particles of said swellable substance have a sphericity S smaller than 2.

8. Precursor according to one of the claims 1 to 7, wherein the said swellable substance is in powdered form.

9. Precursor, according to one of the claim 1 to 8, wherein said discrete particles of said swellable substance have a mean diameter of at least 50 µm.

10. Precursor according to one of the claims 1 to 9, wherein the autoclaving does lead to a decrease of the MW of said swellable substance of minimum 30 %.

11. Precursor according to one of the claims 2 to 10, wherein the autoclaving does lead to a decrease of the MW of said of said swellable substance of maximum 70 %.

12. Precursor according to one of the claims 2 to 11, wherein the autoclaving is performed at a temperature in the range of 110° to 140°C.

13. Precursor according to claim 12, wherein the autoclaving is performed at a temperature in the range of 121°- 128°C.

14. Precursor according to one of the claims 2 to 13, wherein said drying is obtained by the action of dry air, vacuum, freeze-drying and/or a desiccating agent.

15. Precursor according to one of the claims 2 to 14, wherein the loss on drying at 105 °C of said precursor is smaller than 5%.

16. Precursor according to one of the claims 1 to 15, wherein said swellable substance is of biological origin.

17. Precursor according to one of the claims 1 to 15, wherein said swellable substance is of fully synthetic origin.

18. Precursor according to one of the claims 1 to 17, wherein the MW of said swellable substance is larger than 1'000'000 Dalton.

19. Precursor according to claims 1 to 18, wherein the said solid particles have at least a partially porous structure with a pore size between 10 nanometers and 500 micrometers.

20. Precursor according to claim 19, wherein the pore size of said solid particles is between 10 nanometers and 500 micrometers.

21. Precursor according to claim 19 or 20, wherein the porosity of said solid particles is between 60 and 90 percent, preferably between 68 and 84 percent.

22. Precursor according to one of the claims 1 - 6 and 9 - 21, wherein said solid particles comprise a calcium phosphate which is **characterized by** a molar Ca/P relationship between 1.0 and 2.0.

23. Precursor according to one of the claims 1 - 6 and 9 - 22, wherein said solid particles comprise a calcium-phosphate which is **characterized by** a molar Ca/P relationship between 1.45 and 1.52.

24. Precursor according to one of the claim 22 or 23, wherein said calcium phosphate is selected from the following group: Dicalcium phosphate-dihydrate (CaHPO₄ x 2 H₂O), dicalcium phosphate (CaHPO₄) alpha-tricalcium phosphate (alpha-Ca₃(PO)₂), β-tricalcium phosphate (β-Ca₃(PO)₂), calcium-deficient hydroxyapatite (Ca₉(PO₄)₅(HPO₄)OH), hydroxyapatite (Ca₁₀(PO₄)₆OH)₂, carbonated apatite (Ca₁₀(PO₄)₃(CO₃)₃(OH)₂), fluoro apatite (Ca₁₀(PO₄)₆(F,OH)₂), chloro apatite (Ca₁₀(PO₄)₆(Cl,OH)₂), whitlockite ((Ca,Mg)₃(PO₄)₂), tetracalcium phosphate (Ca₄(PO₄)₂O), oxyapatite (Ca₁₀(PO₄)₆O), β-calcium pyrophosphate β-Ca₂(P₂O₇), α-calcium pyrophosphate, gamma-calcium pyrophosphate, and octocalcium phosphate (Ca₈H₂(PO₄)₆ x 5 H₂O), whereby all aforementioned calcium phosphates may be doped with elements such as Na, Cl, F, S, C, Sr, Mg, Zn, Si , Fe, Li, K or Ag.

25. Precursor according to one of the claims 1 - 6 and 9 - 24, wherein said solid particles comprise a mixture of different calcium phosphates.

26. Precursor according to one of the claims 1 - 24, wherein said solid particles comprise a calcium sulfate.

27. Precursor according to one of the claims 1 - 24, wherein said solid particles comprise calcite, aragonite or vaterite which are different polymorphs of calcium carbonate.

28. Precursor according to one of the claims 1 - 6 and 9 - 27, wherein said solid particles comprise a mixture of different calcium phosphates, calcium sulfates, calcium carbonates, and/or calcium-containing bioglass.

29. Precursor according to one of the claims 1 - 28, wherein the specific gravity of said solid particles is between 0.5 and 1.0 g/ccm.

30. Precursor according to one of the claims 1 - 29, wherein said solid particles have a non-spherical shape.

31. Precursor according to one of the claims 1 - 30, wherein said solid particles have a specific surface area (SSA) of larger than 0.01 m²/g.

32. Precursor according to one of the claims 1 - 31, wherein said solid particles have a specific surface area (SSA) of larger than 5m²/g.

33. Precursor according to one of the claims 1 - 32, wherein said discrete particles of said swellable substance have a mean volume of at least 3 ·10⁻⁶ mm³.

34. Precursor according to one of the claims 1 - 33, wherein said discrete particles of said swellable substance have a mean volume of maximum 4.2 mm³.

35. Precursor according to one of the claims 1 - 34, wherein said precursor contains a radiopacifier.

36. Precursor according to claim 35, wherein said radiopacifier is selected from the following group: tantalum powder, tungsten powder, Titanium powder, zirconium oxide powder, bismuth oxide powder, iode-based liquid.

37. Method for preparing the precursor according to one of the claims 1 to 36 comprising the following steps:
a) wet autoclaving of said swellable substance avoiding polymerization of said swellable substance;
b) drying of the product obtained in step "a" avoiding polymerization;
c) mixing the product obtained in step "b" with said solid particles.

38. Bone replacement material obtained by admixing a liquid to the precursor according to one of the claims 1 to 36

39. Bone replacement material according to claim 38, wherein said liquid is pure water, sterile demineralized water, an aqueous solution, a sterile saline solution, sterile Ringer solution, serum, blood, bone marrow an antimicrobial drug solution - preferably an antibiotic solution - or a solution containing osteoinductive substances - preferably bone morphogenetic proteins such as BMP2 and BMP7 or growth factors - and/or drugs against osteoporosis - preferably bisphosphonates and parathyroid hormone.

40. Bone replacement material according to claim 38 or 39, wherein the ratio between the dry weight of the swellable substance and the liquid is in the range of 0.001 and 0.500.

41. Bone replacement material according to claim 40, wherein said ratio is in the range of 0.01 and 0.20.

42. Bone replacement material according to one of the claims 38 - 41, wherein the weight relationship between the hydrated hydrogel and the solid particles is larger than 0.2, preferably larger than 0.6.

43. Bone replacement material according to one of the claims 38 - 42, wherein the weight relationship between the hydrated hydrogel and the solid particles is smaller than 4, preferably smaller than 2.

44. Kit comprising the precursor according to one of the claims 1 - 36 and a liquid suitable for admixing to said precursor in order to convert the resulting mixture into a kneadable mass for bone replacement.

45. Kit according to claim 44, wherein said liquid is pure water, sterile demineralized water, an aqueous solution, a sterile saline solution, sterile Ringer solution, serum, blood, bone marrow, or an antimicrobial drug solution.

## Patentansprüche

1. Vorstufe für die Herstellung eines pastösen Knochenersatzmaterials durch Vermischen einer Flüssigkeit, wobei die Vorstufe Folgendes umfasst:
a) eine biokompatible Substanz, die durch Einwirkung von Wasser oder einer wässrigen Lösung quellbar ist, wodurch ein Hydrogel gebildet wird; und
b) Feststoffpartikel, die aus einer Substanz bestehen, die als Knochenersatzmaterial geeignet ist,
**dadurch gekennzeichnet, dass**
c) die Vorstufe steril für chirurgischen Gebrauch ist;
d) die quellbare Substanz in der Form von diskreten Partikeln ist, die einen mittleren Durchmesser im Bereich von 50 µm bis 1000 µm aufweisen;
e) die Feststoffpartikel und die quellbare Substanz als eine Mischung vorliegen;
f) die Feststoffpartikel keramischer oder mineralischer Natur sind;
g) der mittlere Durchmesser der Feststoffpartikel zwischen 100 und 500 Mikrometern beträgt;
h) das MG der quellbaren Substanz - nach der Sterilisation - größer als 300.000 Dalton beträgt; und
i) die quellbare Substanz Folgendes umfasst:
eine Polyaminosäure oder deren Derivate; oder
ein Polysaccharid und dessen Derivate; vorzugsweise Glycosaminoglycan oder Alginat;
oder
Hyaluronsäure, Natriumhyaluronat, Chondroitinsulfat, Dermatansulfat, Heparansulfat, Heparin oder Keratansulfat.

2. Vorstufe nach Anspruch 1, wobei die Vorstufe durch Nassautoklavierung und anschließendes Trocknen mindestens der quellbaren Substanz gewonnen wurde.

3. Vorstufe nach Anspruch 1 oder 2, wobei die Feststoffpartikel Calcium enthalten.

4. Vorstufe nach einem der Ansprüche 1 bis 3, wobei die quellbare Substanz eine intrinsische Viskosität von mindestens 1,3m³/kg aufweist.

5. Vorstufe nach einem der Ansprüche 1 bis 4, wobei die Feststoffpartikel auf Bioglas(en) basieren.

6. Vorstufe nach einem der Ansprüche 1 bis 5, wobei die diskreten Partikel der quellbaren Substanz eine Sphärizität S kleiner als 5 aufweisen.

7. Vorstufe nach Anspruch 6, wobei die diskreten Partikel der quellbaren Substanz eine Sphärizität S kleiner als 2 aufweisen.

8. Vorstufe nach einem der Ansprüche 1 bis 7, wobei die quellbare Substanz in Pulverform ist.

9. Vorstufe nach einem der Ansprüche 1 bis 8, wobei die diskreten Partikel der quellbaren Substanz einen mittleren Durchmesser von mindestens 50 µm aufweisen.

10. Vorstufe nach einem der Ansprüche 1 bis 9, wobei das Autoklavieren zu einer Abnahme des MG der quellbaren Substanz um mindestens 30 % führt.

11. Vorstufe nach einem der Ansprüche 2 bis 10, wobei das Autoklavieren zu einer Abnahme des MG der quellbaren Substanz von maximal 70 % führt.

12. Vorstufe nach einem der Ansprüche 2 bis 11, wobei das Autoklavieren bei einer Temperatur im Bereich von 110 °C bis 140 °C durchgeführt wird.

13. Vorstufe nach Anspruch 12, wobei das Autoklavieren bei einer Temperatur im Bereich von 121 °C - 128 °C durchgeführt wird.

14. Vorstufe nach einem der Ansprüche 2 bis 13, wobei das Trocknen durch Einwirkung von trockener Luft, Vakuum, Gefriertrocknung und/oder eines Trocknungsmittel erhalten wird.

15. Vorstufe nach einem der Ansprüche 2 bis 14, wobei der Trocknungsverlust bei 105 °C der Vorstufe kleiner als 5 % ist.

16. Vorstufe nach einem der Ansprüche 1 bis 15, wobei die quellbare Substanz biologischen Ursprungs ist.

17. Vorstufe nach einem der Ansprüche 1 bis 15, wobei die quellbare Substanz vollständig synthetischen Ursprungs ist.

18. Vorstufe nach einem der Ansprüche 1 bis 17, wobei das MG der quellbaren Substanz größer als 1.000.000 Dalton ist.

19. Vorstufe nach den Ansprüchen 1 bis 18, wobei die Feststoffpartikel mindestens eine teilweise poröse Struktur mit einer Porengröße zwischen 10 Nanometern und 500 Mikrometern aufweisen.

20. Vorstufe nach Anspruch 19, wobei die Porengröße der Feststoffpartikel zwischen 10 Nanometern und 500 Mikrometern beträgt.

21. Vorstufe nach Anspruch 19 oder 20, wobei die Porosität der Feststoffpartikel zwischen 60 und 90 Prozent beträgt, vorzugsweise zwischen 68 und 84 Prozent.

22. Vorstufe nach einem der Ansprüche 1 - 6 und 9 - 21, wobei die Feststoffpartikel ein Calciumphosphat umfassen, das durch ein molares Ca/P-Verhältnis zwischen 1,0 und 2,0 gekennzeichnet ist.

23. Vorstufe nach einem der Ansprüche 1 - 6 und 9 - 22, wobei die Feststoffpartikel ein Calciumphosphat umfassen, das durch ein molares Ca/P-Verhältnis zwischen 1,45 und 1,52 gekennzeichnet ist.

24. Vorstufe nach einem der Ansprüche 22 oder 23, wobei das Calciumphosphat ausgewählt ist aus der folgenden Gruppe: Dicalciumphosphat-Dihydrat (CaHPO₄ x 2 H₂O), Dicalciumphosphat (CaHPO₄), alpha-Tricalciumphosphat (alpha-Ca₃(PO₄)₂), β-Tricalciumphosphat ((β-Ca₃(PO₄)₂), Calcium-defizienter Hydroxyapatit (Ca₉(PO₄)₅(HPO₄)OH), Hydroxyapatit (Ca₁₀(PO₄)₆OH)₂), Carbonatapatit (Ca₁₀(PO₄)₃(CO₃)₃(OH)₂), Fluorapatit (Ca₁₀(PO₄)₆(F,OH)₂), Chlorapatit (Ca₁₀(PO₄)₆(Cl,OH)₂), Whitlockit ((Ca,Mg)₃(PO₄)₂), Tetracalciumphosphat (Ca₄(PO₄)₂O), Oxyapatit (Ca₁₀(PO₄)₆O), β-Calciumpyrophosphat (β-Ca₂(P₂O₇), α-Calciumpyrophosphat, gamma-Calciumpyrophosphat und Octocalciumphosphat (Ca₈H₂(PO₄)₆ x 5 H₂O), wobei alle vorgenannten Calciumphosphate mit Elementen, wie Na, Cl, F, S, C, Sr, Mg, Zn, Si, Fe, Li, K oder Ag dotiert sein können.

25. Vorstufe nach einem der Ansprüche 1 - 6 und 9 - 24, wobei die Feststoffpartikel ein Gemisch verschiedener Calciumphosphate umfassen.

26. Vorstufe nach einem der Ansprüche 1 - 24, wobei die Feststoffpartikel ein Calciumsulfat umfassen.

27. Vorstufe nach einem der Ansprüche 1 - 24, wobei die Feststoffpartikel Calcit, Aragonit oder Vaterit umfassen, die unterschiedliche Polymorphe von Calciumcarbonat sind.

28. Vorstufe nach einem der Ansprüche 1 - 6 und 9 - 27, wobei die Feststoffpartikel ein Gemisch verschiedener Calciumphosphate, Calciumsulfate, Calciumcarbonate und/oder calziumhaltigem Bioglas umfassen.

29. Vorstufe nach einem der Ansprüche 1 - 28, wobei das spezifische Gewicht der Feststoffpartikel zwischen 0,5 und 1,0 g/ccm beträgt.

30. Vorstufe nach einem der Ansprüche 1 - 29, wobei die Feststoffpartikel eine nichtsphärische Form aufweisen.

31. Vorstufe nach einem der Ansprüche 1 - 30, wobei die Feststoffpartikel eine spezifische Oberfläche (SSA) von größer als 0,01 m²/g aufweisen.

32. Vorstufe nach einem der Ansprüche 1 - 31, wobei die Feststoffpartikel eine spezifische Oberfläche (SSA) von größer als 5 m²/g.

33. Vorstufe nach einem der Ansprüche 1 - 32, wobei die diskreten Partikel der quellbaren Substanz ein mittleres Volumen von mindestens 3 x 10⁻⁶ mm³ aufweisen.

34. Vorstufe nach einem der Ansprüche 1 - 33, wobei die diskreten Partikel der quellbaren Substanz ein mittleres Volumen von maximal 4,2 mm³ aufweisen.

35. Vorstufe nach einem der Ansprüche 1 - 34, wobei die Vorstufe ein Röntgenkontrastmittel enthält.

36. Vorstufe nach Anspruch 35, wobei das Röntgenkontrastmittel ausgewählt ist aus der folgenden Gruppe: Tantalpulver, Wolframpulver, Titanpulver, Zirkoniumoxidpulver, Bismutoxidpulver, Flüssigkeit auf lodbasis.

37. Verfahren zur Herstellung der Vorstufe nach einem der Ansprüche 1 bis 36, umfassend die folgenden Schritte:
a) Nassautoklavieren der quellbaren Substanz unter Vermeidung einer Polymerisation der quellbaren Substanz;
b) Trocknen des in Schritt "a" erhaltenen Produkts unter Vermeidung einer Polymerisation;
c) Mischen des in Schritt "b" erhaltenen Produkts mit den Feststoffpartikeln.

38. Knochenersatzmaterial, erhalten durch Vermischen einer Flüssigkeit mit der nach einem der Ansprüche 1 bis 36 erhaltenen Vorstufe.

39. Knochenersatzmaterial nach Anspruch 38, wobei die Flüssigkeit reines Wasser, steriles entmineralisiertes Wasser, eine wässrige Lösung, eine sterile Kochsalzlösung, sterile Ringer-Lösung, Serum, Blut, Knochenmark, eines antimikrobielle Arzneistofflösung - vorzugsweise eine Antibiotika-Lösung - oder eine Lösung, die osteoinduktive Substanzen enthält - vorzugsweise knochenmorphogenetische Proteine, wie BMP2 und BMP7 oder Wachstumsfaktoren, - und/oder Arzneistoffe gegen Osteoporose - vorzugsweise Bisphosphonate und Parathormon.

40. Knochenersatzmaterial nach Anspruch 38 oder 39, wobei das Verhältnis zwischen dem Trockengewicht der quellbaren Substanz und der Flüssigkeit im Bereich von 0,001 und 0,500 liegt.

41. Knochenersatzmaterial nach Anspruch 40, wobei das Verhältnis im Bereich von 0,01 und 0,20 liegt.

42. Knochenersatzmaterial nach einem der Ansprüche 38 - 41, wobei das Gewichtsverhältnis zwischen dem hydratisierten Hydrogel und den Feststoffpartikeln größer als 0,2 ist, vorzugsweise größer als 0,6.

43. Knochenersatzmaterial nach einem der Ansprüche 38 - 42, wobei das Gewichtsverhältnis zwischen dem hydratisierten Hydrogel und den Feststoffpartikels kleiner als 4 ist, vorzugsweise kleiner als 2.

44. Kit, umfassend die Vorstufe nach einem der Ansprüche 1 - 36 und eine Flüssigkeit, die sich zur Beimischung zu der Vorstufe eignet, um das erhaltene Gemisch in eine knetbare Knochenersatzmasse umzuwandeln.

45. Kit nach Anspruch 44, wobei die Flüssigkeit reines Wasser, steriles entmineralisiertes Wasser, eine wässrige Lösung, eine sterile Kochsalzlösung, sterile Ringer-Lösung, Serum, Blut, Knochenmark oder eine antimikrobielle Arzneistofflösung ist.

## Revendications

1. Précurseur pour la préparation d'un matériau pâteux de remplacement osseux par mélange d'un liquide, ledit précurseur comprenant
a) une substance biocompatible pouvant gonfler par l'action de l'eau ou d'une solution aqueuse, formant ainsi un hydrogel ; et
b) des particules solides faites d'une substance qui convient comme matériau de remplacement osseux,
**caractérisé en ce que**
c) le précurseur est stérile pour une utilisation chirurgicale ;
d) ladite substance pouvant gonfler se présente sous la forme de particules discrètes ayant un diamètre moyen dans la plage de 50 µm à 1 000 µm;
e) lesdites particules solides et ladite substance pouvant gonfler sont présentes sous forme de mélange ;
f) lesdites particules solides sont de nature céramique ou minérale ;
g) le diamètre moyen desdites particules solides est compris entre 100 et 500 micromètres ;
h) la MM de ladite substance pouvant gonfler est - après stérilisation - plus grande que 300 000 Daltons ; et
i) ladite substance pouvant gonfler comprend :
un polyaminoacide ou ses dérivés ; ou
un polysaccharide et leurs dérivés ; de préférence le glycosaminoglycane
ou l'alginate ; ou
l'acide hyaluronique, le hyaluronate de sodium, la chondroïtine sulfate, le dermatane sulfate, l'héparane sulfate, l'héparine ou le kératane sulfate.

2. Précurseur selon la revendication 1, dans lequel ledit précurseur a été obtenu par autoclavage humide et séchage ultérieur de ladite substance pouvant gonfler.

3. Précurseur selon la revendication 1 ou 2, dans lequel lesdites particules solides contiennent du calcium.

4. Précurseur selon l'une des revendications 1 à 3, dans lequel ladite substance pouvant gonfler a une viscosité intrinsèque d'au moins 1,3 m³/kg.

5. Précurseur selon l'une des revendications 1 à 4, dans lequel lesdites particules solides sont à base de verre(s) biologique(s).

6. Précurseur selon l'une des revendications 1 à 5, dans lequel lesdites particules discrètes de ladite substance pouvant gonfler ont une sphéricité S plus petite que 5.

7. Précurseur selon la revendication 6, dans lequel lesdites particules discrètes de ladite substance pouvant gonfler ont une sphéricité S plus petite que 2.

8. Précurseur selon l'une des revendications 1 à 7, dans lequel ladite substance pouvant gonfler est sous forme de poudre.

9. Précurseur selon l'une des revendications 1 à 8, dans lequel lesdites particules discrètes de ladite substance pouvant gonfler ont un diamètre moyen d'au moins 50 µm.

10. Précurseur selon l'une des revendications 1 à 9, dans lequel l'autoclavage conduit bien à une diminution de la MM de ladite substance pouvant gonfler de 30 % minimum.

11. Précurseur selon l'une des revendications 2 à 10, dans lequel l'autoclavage conduit bien à une diminution de la MM de ladite substance pouvant gonfler de 70 % maximum.

12. Précurseur selon l'une des revendications 2 à 11, dans lequel l'autoclavage est réalisé à une température dans la plage de 110°C à 140°C.

13. Précurseur selon la revendication 12, dans lequel l'autoclavage est réalisé à une température dans la plage de 121°C à 128°C.

14. Précurseur selon l'une des revendications 2 à 13, dans lequel ledit séchage est obtenu par l'action de l'air sec, du vide, d'une lyophilisation et/ou d'un agent de dessiccation.

15. Précurseur selon l'une des revendications 2 à 14, dans lequel la perte au séchage à 105 °C dudit précurseur est plus petite que 5 %.

16. Précurseur selon l'une des revendications 1 à 15, dans lequel ladite substance pouvant gonfler est d'origine biologique.

17. Précurseur selon l'une des revendications 1 à 15, dans lequel ladite substance pouvant gonfler est d'origine totalement synthétique.

18. Précurseur selon l'une des revendications 1 à 17, dans lequel la MM de ladite substance pouvant gonfler est plus grande que 1 000 000 Daltons.

19. Précurseur selon les revendications 1 à 18, dans lequel lesdites particules solides ont une structure au moins partiellement poreuse avec une taille de pore entre 10 nanomètres et 500 micromètres.

20. Précurseur selon la revendication 19, dans lequel la taille de pore desdites particules solides est comprise entre 10 nanomètres et 500 micromètres.

21. Précurseur selon la revendication 19 ou 20, dans lequel la porosité desdites particules solides est comprise entre 60 et 90 pour cent, de préférence entre 68 et 84 pour cent.

22. Précurseur selon l'une des revendications 1 à 6 et 9 à 21, dans lequel lesdites particules solides comprennent un phosphate de calcium qui est **caractérisé par** une relation Ca/P molaire comprise entre 1,0 et 2,0.

23. Précurseur selon l'une des revendications 1 à 6 et 9 à 22, dans lequel lesdites particules solides comprennent un phosphate de calcium qui est **caractérisé par** une relation Ca/P molaire comprise entre 1,45 et 1,52.

24. Précurseur selon l'une des revendications 22 ou 23, dans lequel ledit phosphate de calcium est choisi dans le groupe suivant : le phosphate dicalcique dihydraté (CaHPO₄ x 2 H₂O), le phosphate dicalcique (CaHPO₄), le phosphate tricalcique alpha (alpha-Ca₃(PO₄)₂), le phosphate tricalcique β (β-Ca₃(PO₄)₂), l'hydroxyapatite déficiente en calcium (Ca₉(PO₄)₅(HPO₄)OH), l'hydroxyapatite (Ca₁₀(PO₄)₆OH)₂), l'apatite carbonatée (Ca₁₀(PO₄)₃(CO₃)₃(OH)₂), la fluorapatite (Ca₁₀(PO₄)₆(F,OH)₂), la chlorapatite (Ca₁₀(PO₄)₆(Cl,OH)₂), la whitlockite ((Ca, Mg)₃(PO₄)₂), le phosphate tétracalcique (Ca₄(PO₄)₂O), l'oxyapatite (Ca₁₀(PO₄)₆O), le pyrophosphate de calcium β (β-Ca₂(P₂O₇), le pyrophosphate de calcium α, le pyrophosphate de calcium gamma, et le phosphate octocalcique (Ca₈H₂(PO₄)₆ x 5 H₂O), moyennant quoi tous les phosphates de calcium précités peuvent être dopés avec des éléments tels que Na, CI, F, S, C, Sr, Mg, Zn, Si, Fe, Li, K ou Ag.

25. Précurseur selon l'une des revendications 1 à 6 et 9 à 24, dans lequel lesdites particules solides comprennent un mélange de phosphates de calcium différents.

26. Précurseur selon l'une des revendications 1 à 24, dans lequel lesdites particules solides comprennent un sulfate de calcium.

27. Précurseur selon l'une des revendications 1 à 24, dans lequel lesdites particules solides comprennent de la calcite, de l'aragonite ou de la vatérite qui sont des polymorphes différents de carbonate de calcium.

28. Précurseur selon l'une des revendications 1 à 6 et 9 à 27, dans lequel lesdites particules solides comprennent un mélange de différents phosphates de calcium, sulfates de calcium, carbonates de calcium, et/ou de verre biologique contenant du calcium.

29. Précurseur selon l'une des revendications 1 à 28, dans lequel la densité desdites particules solides est comprise entre 0,5 et 1,0 g/ccm.

30. Précurseur selon l'une des revendications 1 à 29, dans lequel lesdites particules solides ont une forme non sphérique.

31. Précurseur selon l'une des revendications 1 à 30, dans lequel lesdites particules solides ont une aire de surface spécifique (ASS) de plus de 0,01 m²/g.

32. Précurseur selon l'une des revendications 1 à 31, dans lequel lesdites particules solides ont une aire de surface spécifique (ASS) de plus de 5 m²/g.

33. Précurseur selon l'une des revendications 1 à 32, dans lequel lesdites particules discrètes de ladite substance pouvant gonfler ont un volume moyen d'au moins 3·10⁻⁶ mm³.

34. Précurseur selon l'une des revendications 1 à 33, dans lequel lesdites particules discrètes de ladite substance pouvant gonfler ont un volume moyen de 4,2 mm³ maximum.

35. Précurseur selon l'une des revendications 1 à 34, dans lequel ledit précurseur contient un radio-opacifiant.

36. Précurseur selon la revendication 35, dans lequel ledit radio-opacifiant est choisi dans le groupe suivant : poudre de tantale, poudre de tungstène, poudre de titane, poudre d'oxyde de zirconium, poudre d'oxyde de bismuth, liquide à base d'iode.

37. Procédé de préparation du précurseur selon l'une des revendications 1 à 36, comprenant les étapes suivantes :
a) autoclavage humide de ladite substance pouvant gonfler en évitant une polymérisation de ladite substance pouvant gonfler ;
b) séchage du produit obtenu à l'étape « a » en évitant une polymérisation ;
c) mélange du produit obtenu à l'étape « b » avec lesdites particules solides.

38. Matériau de remplacement osseux obtenu par mélange d'un liquide avec le précurseur selon l'une des revendications 1 à 36.

39. Matériau de remplacement osseux selon la revendication 38, dans lequel ledit liquide est de l'eau pure, de l'eau déminéralisée stérile, une solution aqueuse, une solution saline stérile, la solution de Ringer stérile, du sérum, du sang, de la moelle osseuse, une solution de médicament antimicrobienne-de préférence une solution antibiotique - ou une solution contenant des substances d'ostéoinduction - de préférence des protéines morphogénétiques osseuses telles que BMP2 et BMP7 ou des facteurs de croissance - et/ou des médicaments contre l'ostéoporose - de préférence des bisphosphonates et la parathormone.

40. Matériau de remplacement osseux selon la revendication 38 ou 39, dans lequel le rapport entre le poids sec de la substance pouvant gonfler et le liquide est dans la plage de 0,001 à 0,500.

41. Matériau de remplacement osseux selon la revendication 40, dans lequel ledit rapport est dans la plage de 0,01 à 0,20.

42. Matériau de remplacement osseux selon l'une des revendications 38 à 41, dans lequel la relation en poids entre l'hydrogel hydraté et les particules solides est plus grande que 0,2, de préférence plus grand que 0,6.

43. Matériau de remplacement osseux selon l'une des revendication 38 à 42, dans lequel la relation en poids entre l'hydrogel hydraté et les particules solides est plus petite que 4, de préférence plus petit que 2.

44. Kit comprenant le précurseur selon l'une des revendications 1 à 36, et un liquide convenant au mélange avec ledit précurseur afin de convertir le mélange résultant en une masse malaxable pour remplacement osseux.

45. Kit selon la revendication 44, dans lequel ledit liquide est de l'eau pure, de l'eau déminéralisée stérile, une solution aqueuse, une solution saline stérile, une solution de Ringer stérile, du sérum, du sang, de la moelle osseuse, ou une solution de médicament antimicrobienne.
